# EUROPEAN PATENT APPLICATION

(11) **EP 1 595 546 A1**
(43) Date of publication of application: **16.11.2005**
(21) Application number: 03815859.8
(22) Date of filing: 10.11.2003
(51) Int. Cl.: A61K 35/12, A61P 35/00

(54) **REMEDY FOR CANCER**

(30) Priority: 17.02.2003 JP 2003038117; 18.02.2003 JP 2003039122
(71) Applicant: Mebiopharm Co., Ltd., Tokyo 107-0052 (JP)
(72) Inventor: UTOGUCHI, Naoki, Sagamihara-shi, Kanagawa 229-1103 (JP); MARUYAMA, Kazuo, Sagamihara-shi, Kanagawa 229-1101 (JP)
(74) Representative: Hartz, Nikolai
(86) International application number: PCT/JP2003/014252
(87) International publication number: WO 2004/071518

(57) **Abstract**

The present invention is directed to a therapeutic drug for cancer, containing, as an active ingredient, dendritic cells stimulated with an antigen derived from vascular endothelial cells which have been cultured in a medium containing a cancer cell culture supernatant.

## Description

### TECHNICAL FIELD

The present invention relates to cancer immunotherapy targeting vascular endothelial cells of tumor tissue.

### BACKGROUND ART

A variety of approaches have been followed for treatment of cancers, including surgical therapy, chemotherapy, and radiotherapy. Each of these approaches has its own merits, and therefore, after considering their characteristics, practitioners select a method that is considered to be the most effective against the cancer to be treated. Surgical therapy is an effective method for cancerous tissues having a certain size or more. However, surgical invasion causes patients to suffer great pain and discomfort, and in addition, has some limitations. For example, there are cases where the lesion site is in a location difficult to access for removal, as in the case of brain tumors. Also, small cancers having a size of several millimeters are difficult to locate. Chemotherapy is primarily performed through injection or oral administration, which are less burdensome to patients. Moreover, since anticancer drugs are delivered via blood flow, small tumors can be treated. However, anticancer drugs are essentially toxic to cells, producing extremely severe side effects. Restrictions on dosage and administration period of such drugs often result in unsatisfactory results. In radiotherapy, pin-pointed irradiation of the target site with radioactive rays is sometimes difficult, and in addition, surrounding healthy cells are also affected by such irradiation.

As described above, each of these therapeutic methods has its strong points and weak points. Therefore, in many cases, these therapies are not performed solely but are combined appropriately for the treatment of cancer. Yet, cancer remains as the leading cause of death in Japan, and therefore, establishment of a novel cancer therapeutic method is a pressing need.

In addition to the above three conventional cancer therapies, there is a fourth approach; namely, cancer immunotherapy. This approach aims to eliminate cancer through the immune system, which is a natural system intrinsically possessed by the living subjects for expelling alien substances. In early cancer immunotherapy, a cancer vaccine was prepared from disrupted cancer cells or a cancer-specific antigen, along with an adjuvant or a similar material, and the vaccine was administered to a subject with an aim to activate cytotoxic T lymphocytes (CTLs). Although this was expected to achieve successful elimination of cancerous tissue, in reality, virtually no favorable results have been obtained therefrom, as cancer cells per se have been derived from the patient's own cells and thus have very low antigenicity.

In recent years, roles of dendritic cells have been elucidated, and have attracted attention as providing a breakthrough for novel cancer immunotherapy. Among different types of biological cells, dendritic cells exhibit the strongest antigen-presenting ability in a living organism. In such a dendritic-cell-based immunotherapy, dendritic cells are pulsed with crushed cancer cell fragments or a cancer specific antigen in an ex vivo system, and the resultant dendritic cells are returned to a living subject, where the dendritic cells activate CTLs and the immunization system of the subject destroys cancerous tissue. Such cancer immunotherapy making use of dendritic cells not only has been performed on an animal experiment basis, but successful results have been reported in human clinical settings. Thus, the dendritic-cell-based immunotherapy has become of interest for its potential and effectiveness as a promising novel cancer therapy (J. Immunol., 156, p. 2918 (1996), J. Exp. Med., 183, p. 7 (1996), Blood, 84, p. 3054 (1994), Immunol., 95, p. 141 (1998), J. Exp. Med., 185, p. 1101 (1997), J. Exp. Med., 187, p. 1019 (1998), Blood, 93, p. 780 (1999), Science, 283, p. 1183 (1999), J. Exp. Med, 185, p. 1101 (1997)).

### DISCLOSURE OF THE INVENTION

An object of the present invention is to provide a novel cancer immunotherapy making use of the functions of dendritic cells.

The present inventors, realizing that tumor tissue grows at a higher cell proliferation rate than does healthy tissue and thus inevitably requires neovascularization to secure the pathway for enabling the supply of nutrients and discharge of wastes therethrough, have found that dendritic cells which are useful in the production of a drug for cancer immunotherapy targeting vascular endothelial cells of tumor tissue can be obtained through incubation of vascular endothelial cells in a medium containing a supernatant of the cancer cell culture, followed by stimulation of the dendritic cells with the thus-created vascular-endothelial-cell-derived antigen. The present invention has been achieved on a basis of this finding.

Accordingly, the present invention provides a therapeutic drug for cancer containing, as an active ingredient, dendritic cells stimulated with an antigen derived from vascular endothelial cells which have been cultured in a medium containing a cancer cell culture supernatant.

The present invention also provides use, in the production of a therapeutic drug for cancer, of dendritic cells stimulated with an antigen derived from vascular endothelial cells which have been cultured in a medium containing a cancer cell culture supernatant.

The present invention also provides a therapeutic method for cancer, characterized by administering dendritic cells stimulated with an antigen derived from vascular endothelial cells which have been cultured in a medium containing a cancer cell culture supernatant.

The cancer remedy according to the present invention belongs to a cancer immunotherapy targeting not tumor cells themselves but vascular endothelial cells of tumor tissue. Therefore, the present invention has the following advantages over conventional types of cancer immunotherapy.
(1) The ratio between the two types of cells forming a mass of tumor tissue is cancer cells : vascular endothelial cells = 100 to 1000 : 1, which means that a single vascular endothelial cell supports 100 to 1000 cancer cells. Therefore, one death incidence of a vascular endothelial cell is expected to lead to the death of 100 to 1000 cancer cells. It follows that if vascular endothelial cells are targeted, a 100- to 1000-fold efficiency would be obtained as compared with the case where cancer cells themselves are targeted.
(2) According to the conventional cancer immunotherapy, in the case of treatment of primary carcinoma, therapeutic effect can be expected only when immunization is carried out by use of cells specific to the cancer species of interest; i.e., liver cancer cells for the treatment of liver cancer, and lung cancer cells for the treatment of lung cancer. However, when vascular endothelial cells are targeted, the above problem can be avoided; irrespective of whether the cancer to be treated is, for example, liver cancer or lung cancer, antigens are derived from vascular endothelial cells, and so long as such vascular endothelial cells have been under influence of cancer cells, the cells have common characteristics irrespective of cancer species and express common antigen, proving validity of a single remedy for any cancer species.
(3) In the case of an adult, other than tumor tissues, the only site where active neovascularization takes place in the living body is the site of a wound which is in the healing process, and therefore, when cancerous vascular endothelial cells are targeted, very high tumor selectivity is expected, with reduced side effects.

### BRIEF DESCRIPTION OF THE DRAWINGS

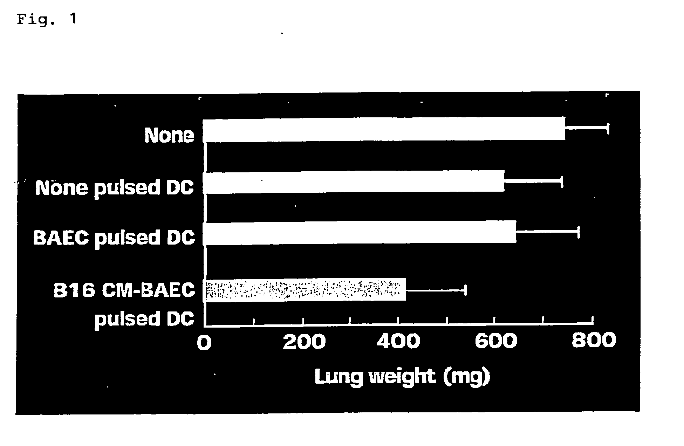

Fig. 1 is a graph showing the therapeutic effect, on mouse lung cancer, of dendritic cells stimulated with an antigen derived from vascular endothelial cells which have been cultured in a medium containing a B16 cancer cell culture supernatant, wherein the effect is shown on the basis of lung weight. In this Figure, the "None" group indicates the case where B16 alone was administered; the "None pulsed DC" group indicates the case where B16 and dendritic cells which had not been pulsed were administered; the "BAEC pulsed DC" group indicates the case where BAEC-pulsed dendritic cells were administered; and the "B16 CM-BAEC pulsed DC" indicates the case where B16 and dendritic cells which had been pulsed with BAEC cultured in a B16 culture supernatant were administered.

### BEST MODE FOR CARRYING OUT THE INVENTION

The dendritic cells serving as an active ingredient of the cancer therapeutic agent of the present invention take vascular endothelial cells of tumor tissue as their targets. For this reason, the vascular endothelial cells to be used for stimulating dendritic cells are those which have been cultured in a medium containing a cancer cell culture supernatant.

The cancer cell is preferably a human cancer cell. However, no particular limitation is imposed on the species of the cancer cell. Examples of the cancer cell includes cells of solid cancer, such as gastric cancer, liver cancer, colon cancer, lung cancer, skin cancer, bladder cancer, uterine cervical cancer, ovarian cancer, breast cancer, prostatic cancer, and brain tumor. No particular limitation is imposed on the culture conditions under which the cancer cell culture is performed. For example, culturing is preferably performed in a medium such as RPMI 1640, MEM, DMEM, Ham's F12, or M199, at 37°C, in an atmosphere of 5% CO₂ and 95% air, under saturated water vapor conditions. The medium may be supplemented with an antibiotic, sodium hydrogencarbonate, a non-essential amino acid, or another additive. The supernatant of a cancer cell culture may be collected through centrifugation performed, for example, at 500 to 1,500 rpm for 2 to 10 minutes, and filtration.

No particular limitation is imposed on the species of the source of vascular endothelial cells. For example, vascular endothelial cells may be collected from a human, bovine, or a mouse. Examples of the human source for providing vascular endothelial cells include umbilical cord vein, umbilical cord artery, aorta, pulmonary artery, and blood vessels of adult skin and neonatal foreskin. Culturing of such vascular endothelial cells is performed in a medium containing the aforementioned cancer cell culture supernatant. The volume of the cancer cell culture supernatant to be added to the medium is preferably 10 to 100 v/v%, more preferably 20 to 100 v/v%. No particular limitation is imposed on the medium in which vascular endothelial cells are cultured. Examples of the medium include, but are not limited to, RPMI 1640, MEM, DMEM, Ham's F12, and M199, and any of these may be supplemented with, other than a cancer cell culture supernatant, vascular endothelial cell growth factor, heparin, an antibiotic, or a similar substance. No particular limitation is imposed on the culture conditions under which vascular endothelial cells are cultured, but preferably, culturing is performed at 37°C, in an atmosphere of 5% CO₂ and 95% air, under saturated water vapor conditions for 2 to 4 days.

Examples of antigens derived from the aforementioned vascular endothelial cells include vascular endothelial cells *per se,* intracellular components of vascular endothelial cells (e.g., total RNA), vascular endothelial cell lysates, and vascular endothelial cell membrane vesicles. Intracellular components, cell lysates, and membrane vesicles are preferred, as they are easy to prepare and effectively stimulate dendritic cells.

A cell lysate may be prepared through, for example, low-speed centrifugation (400 rpm for 10 minutes) of a cell product which has been prepared by subjecting cells to 4 to 6 cycles of freezing (-160°C) and thawing (37°C). Also, a membrane vesicle may be prepared, for example, through the following process. Briefly, cells are treated overnight with a mixture containing DMEM and, as additives, 100 mM p-formaldehyde, 2 mM dithiothreitol, 1 mM CaCl₂, and 0.5 mM MgCl₂ at 37°C in an atmosphere of 5% CO₂ and 95% air, under saturated water vapor conditions, followed by centrifugation for 5 minutes at 150 × g. Afterwards, the supernatant is centrifuged for 30 minutes at 30,000 × g at 4°C. Intracellular components, such as total RNA, may be prepared through a routine method.

The dendritic cells are preferably of human origin, and particularly preferably are collected from the patient to whom the drug of the present invention is to be administered, or from a human who is MHC compatible with the patient. Dendritic cells may be obtained from, for example, myeloma cells, umbilical-cord-blood-derived cells, or peripheral mononuclear cells, through differentiation induced in accordance with, for example, a method described in a non-patent document 8 or 9. In order to obtain dendritic cells from peripheral mononuclear cells by inducing differentiation, preferably, incubation is performed in a medium supplemented with GM-CSF (10 to 100 ng/mL) and IL-4 (50 to 500 IU/mL) for 5 to 10 days.

Stimulation of dendritic cells with the above-described antigens derived from vascular endothelial cells may be performed, for example, through addition of the vascular-endothelial-cell-derived antigens to a suspension of dendritic cells, and incubation of the mixture for 4 to 24 hours. Preferably, cationic liposome or a similar substance is also used in combination, to thereby improve the efficiency of stimulation with antigens. Furthermore, cell fusion of dendritic cells and vascular endothelial cells may be performed, so that vascular endothelial cell antigens are presented by the dendritic cells. Alternatively, total RNA of vascular endothelial cells may be introduced into dendritic cells, to thereby induce presentation of antigens. Preferably, the antigens are added in an amount of 0.01 to 100 µg/mL, more preferably 0.1 to 100 µg/mL, on a protein concentration basis per 1×10⁶ dendritic cells.

The thus-obtained dendritic cells are useful as a cancer remedy by virtue of exerting the following functions. Briefly, they present antigens specific for vascular endothelial cells of a cancer tissue in the living organism, whereby cytotoxic T lymphocytes (CTLs) reactive against vascular endothelial cells of the tumor tissue are induced. Thus, the vascular endothelial cells of the tumor tissue are attacked, and the blood vessels in the tumor tissue are destroyed. As a result, nutrition supply is cut, leading to retraction of the tumor.

In view that the cell suspension containing the above-prepared dendritic cells is administered to humans as a therapeutic drug for cancer, the suspension is preferably subjected to a process for eliminating cell proliferative properties. For safer use of the drug, the dendritic-cell-containing suspension may be, for example, heated, radiated, or treated with mitomycin C, under mild conditions under which only cancer cell protein is denatured while the function of the dendritic cells as a therapeutic drug for cancer is retained. In the case where X-ray irradiation is performed, for example, a flask containing the dendric cells is placed under the tube of an X-ray radiation apparatus, and the flask is irradiated with X-rays at a total radiation dose of 1,000 to 3,300 Rad. In the case where mitomycin C treatment is performed, for example, to a suspension containing the dendritic cells at a cell density of 1×10⁷ to 3×10⁷ cells/mL, mitomycin C is added in a ratio of 25 to 50 µg per mL of cell suspension, and the resultant mixture is left to stand at 37°C for 30 to 60 minutes. In the case where heat treatment is performed, for example, a centrifuge tube containing a cell suspension prepared to have a live cell concentration of 1×10⁷ cells/mL is heated at 50 to 65°C for 20 minutes.

No particular limitation is imposed on the type of cancer to be treated by the cancer therapeutic drug of the present invention. However, a solid cancer is preferred. Examples of the solid cancer include gastric cancer, liver cancer, colon cancer, lung cancer, skin cancer, bladder cancer, uterine cervical cancer, ovarian cancer, breast cancer, prostate cancer, and brain tumor. The amount of administration of the dendritic cells of the present invention varies depending on, for example, patient's age, body weight, and sex, as well as the identity and the stage of cancer, symptoms or the like, and cannot be determined uniformly. The dendritic cells of the present invention may be administered to a patient in about the same amount as the injection amount employed in the current cell vaccine therapy. The dendritic cells of the present invention may be administered to a single intended patient, or alternatively, thanks to the well-established network of bone marrow banks and umbilical cord blood banks, they may be administered to numerous other patients having MHC compatibility.

### EXAMPLES

The present invention will next be described in more detail by way of Examples, which should not be construed as limiting the invention thereto.

### Example 1

### A. Method

### (1) Preparation of cancer cell culture supernatant (cancer CM)

Mouse melanoma B16 cells were cultured in RPMI 1640 medium at 37°C in an atmosphere of 5% CO₂ and 95% air under saturated water vapor conditions until subconfluency. The medium was replaced with a fresh medium (a culture medium for human umbilical cord vein endothelial cells, hereinafter referred to as HUVEC; composed of a mixture of Medium 199 and RPMI 1640 in the ratio of 1 : 1 and supplemented with 15% fetal calf serum, an endothelial cell proliferation factor (20 µg/mL), heparin (25 µg/mL), and an antibiotic). After 48 hours, the medium was recovered and subjected to centrifugation at 1,000 rpm for 5 minutes. The supernatant was filtered by use of a 0.22 µm filter. The filtrate and a fresh HUVEC medium were mixed in equal volumes, and the mixture was used as a conditioned medium for cancer cells (cancer CM).

### (2) Preparation of cancer-CM-reacted vascular endothelial cells

The human umbilical cord vein endothelial cells were seeded in the HUVEC medium at a concentration of 2,500 cells/cm², and cultured at 37°C in an atmosphere of 5% CO₂ and 95% air under saturated water vapor conditions. On the next day, the medium was replaced with the cancer CM. The umbilical cord vein endothelial cells were cultured in this medium at 37°C in an atmosphere of 5% CO₂ and 95% air under saturated water vapor conditions for 48 hours, and the cultured cells were used as cancer-CM-reacted vascular endothelial cells.

### (3) Antigen for pulsing dendritic cells

A membrane vesicle preparatory solution (DMEM in which 100 mM p-formaldehyde, 2 mM dithiothreitol, 1 mM CaCl₂, and 0.5 mM MgCl₂ were dissolved) was added to the cultured cancer-CM-reacted vascular endothelial cells for treatment of the cells overnight at 37°C.

The supernatant was then subjected to centrifugation at 150×g for 5 minutes. Subsequently, the supernatant was subjected to centrifugation for 30 minutes at 4°C and 30,000xg. The resultant pellet was used as membrane vesicles of the cancer-CM-reacted vascular endothelial cells for serving as an antigen for pulsing the dendritic cells.

Separately, cultured cancer-CM-reacted.vascular endothelial cells were dissolved in saline to form a suspension, and the suspension was subjected to four cycles of freezing and thawing. The cell lysate was also used as an antigen for pulsing the dendritic cells.

The membrane vesicles of the cancer-CM-reacted vascular endothelial cells or the cell lysate were mixed with lipofectin (on a volume basis) so as to attain a lipid concentration of 10 µg/mL, and the mixture was left to stand at room temperature for 20 minutes. The mixture was added to a cell suspension (1×10⁶ cells/mL) of mouse dendritic cell strain DC2.4 cells or bone-marrow-derived, primary-cultured dendritic cells, and the cells were incubated for 5 hours. The resultant cells were subjected to centrifugation using a phosphate buffer. After washing, the cells were treated with mitomycin C (50 µg/mL) at 37°C for 30 minutes. Subsequently, the cells were subjected to centrifugation using the phosphate buffer twice, washed, and re-suspended in phosphate buffer.

### (4) Administration of dendritic cells

The thus-prepared DC2.4 cells or the primary-cultured dendritic cells were administered subcutaneously or intracutaneously in an amount of 1×10⁵ cells to B57/BL6 mice.

### (5) Administration of cancer cells

One week after the administration of the DC2.4 cells or the primary-cultured dendritic cells, B16F10 cells (1×10⁶ cells) were injected into the tail vein.

### (6) Evaluation of lung metastasis incidence

One week after the administration of the B16F10 cells, the lung was removed, and the number of colonies which had spread to the lung was counted under a stereoscopic microscope.

### B. Results

The lung metastasis incidences of three mouse cases to which the dendritic cells had not been administrated were (924, 799, 550), and the lung metastasis incidences of three mouse cases to which the dendritic cells had been administrated were (184, 414, 0). The dendritic cells significantly suppressed the lung metastasis. The results have proven that the dendritic cells of the present invention are useful as a therapeutic drug for cancer.

### Example 2

### A. Method

Bovine aorta endothelial cells (BAEC) which had been scraped off from the intima and subjected to subculture were employed. The medium employed for culturing the endothelial cells is DMEM supplemented with 10% fetal calf serum. The endothelial cells were processed in the same manner as in Example 1, to thereby produce cell lysate serving as an antigen.

Through the same procedure as in Example 1, except that a medium containing a supernatant of B16 cell culture was used, the bovine-aorta-derived endothelial cells were cultured. The dendritic cells were pulsed with the cell lysate of the cultured cells, which served as an antigen. The pulsed dendritic cells were administered to B57/BL6 mice by the same method as in Example 1. Two weeks after the cancer cell administration, the lung was removed, and the lung weight was measured.

### B. Results

As shown in Fig. 1, in the cases of the "None" group (administration of B16 only), the "None pulsed DC" group (administration of B16 and the non-pulsed dendritic cells), and the "BAEC pulsed DC" group (administration of B16 and the BAEC pulsed dendritic cells), almost all portions of the lung were found to be black due to B16, and the lung weight was also found to be increased. In contrast to the above, in the case of the "B16CM-BAEC pulsed DC" group (administration of B16 and the dendritic cells which had been pulsed with BAEC cultured in B16 culture supernatant), the black areas in the lung were found to be very limited, and an increase in the lung weight was also found to be suppressed.

## Claims

1. A therapeutic drug for cancer, comprising, as an active ingredient, dendritic cells stimulated with an antigen derived from vascular endothelial cells which have been cultured in a medium containing a cancer cell culture supernatant.

2. The therapeutic drug for cancer according to claim 1, wherein the antigen derived from vascular endothelial cells comprises a vascular endothelial cell, an intracellular component of a vascular endothelial cell, a vascular endothelial cell lysate, or a vascular endothelial cell membrane vesicle.

3. Use, in production of a therapeutic drug for cancer, of dendritic cells stimulated with an antigen derived from vascular endothelial cells which have been cultured in a medium containing a cancer cell culture supernatant.

4. The use according to claim 3, wherein the antigen derived from vascular endothelial cells comprises a vascular endothelial cell, an intracellular component of a vascular endothelial cell, a vascular endothelial cell lysate, or a vascular endothelial cell membrane vesicle.

5. A therapeutic method for cancer, which comprises administering dendritic cells stimulated with an antigen derived from vascular endothelial cells which have been cultured in a medium containing a cancer cell culture supernatant.

6. The therapeutic method according to claim 5, wherein the antigen derived from vascular endothelial cells comprises a vascular endothelial cell, an intracellular component of a vascular endothelial cell, a vascular endothelial cell lysate, or a vascular endothelial cell membrane vesicle.
